# EUROPÄISCHE PATENTSCHRIFT

(11) **EP 1 447 062 B1**
(45) Veröffentlichungstag und Bekanntmachung des Hinweises auf die Patenterteilung: **24.05.2006**
(21) Anmeldenummer: 03028600.9
(22) Anmeldetag: 12.12.2003
(51) Int. Cl.: A61F 2/60, A61F 2/68

(54) **Beinprothese**
Leg prosthesis
Prothèse de jambe

(30) Priorität: 17.02.2003 DE 10307328
(43) Veröffentlichungstag der Anmeldung: 18.08.2004
(73) Patentinhaber: ESKA Implants GmbH & Co., 23556 Lübeck (DE)
(72) Erfinder: Grundei, Hans Dr.-Ing., 23558 Lübeck (DE)
(74) Vertreter: Fuchs Mehler Weiss & Fritzsche

(56) Entgegenhaltungen:
- EP-A- 0 358 056
- EP-A- 0 549 855
- WO-A-00/38599
- WO-A-01/43669
- WO-A-99/08621
- DE-A1- 19 953 972
- US-A- 5 133 773
- US-A- 6 113 642

## Beschreibung

Die vorliegende Erfindung betrifft eine Beinprothese zur Adaption an einen Oberschenkelstumpf, wie sie aus der DE-A-199 53 972 bekannt ist. Diese besteht aus einem Adapter für ein Kniegelenk, welcher mit einem Stielteil im Femurstumpf des Patienten verankert wird. Distal tritt der Adapter aus dem Oberschenkelstumpf aus und bietet dort eine Ankopplungsmöglichkeit für ein künstliches Kniegelenk, wie es beispielsweise bekannt ist aus der EP-B-0 358 056.

An das Kniegelenk ist ein Prothesenunterschenkel angekoppelt, welcher seinerseits distal einen angelenkten Prothesenfuß trägt. Dieser lässt sich von einer Spitzfußstellung in eine Hackenfußstellung schwenken.

Das Kniegelenk gemäß dem Vorschlag der EP-B-0 358 056 ist so ausgebildet, dass es beim Übergang von der Strecklage in die Beugelage um eine Schwenkachse eine kombinierte Roll- und Gleitbewegung ausführt. Im Gegensatz zu einem reinen Scharniergelenk ist das Kniegelenk der gattungsgemäßen Beinprothese so ausgebildet, dass sich der Abstand eines dorsal gesehen vor der Schwenkachse gelegenen Punktes des Kniegelenks zum Ende des Prothesenunterschenkels stetig verkleinert. Mit anderen Worten vergrößert sich der Abstand eines dagegen ventral gesehen vor der Schwenkachse gelegenen Punktes zum Ende des Prothesenunterschenkels stetig.

Ein Problem für Patienten mit teilamputiertem Oberschenkel ist neben anderen, dass er beim Gehen mit dem gesunden Fuß in eine stärkere Spitzfußstellung gehen muss, um den Prothesenfuß beim erneuten Schritt nach vorn mit der Beinprothese durchpendeln zu lassen. Dies gilt unabhängig davon, ob der Prothesenfuß an dem Prothesenunterschenkel nun verschwenkbar oder aber fest arretiert ist. Die Notwendigkeit, den gesunden natürlichen Fuß in eine verstärkte oder extreme Spitzfußstellung zu bringen, damit die Prothese durchpendeln kann, bedingt eine recht unphysiologische Bewegungsweise und damit einhergehend eine starke Beanspruchung der Wirbelsäule.

Der gattungsgemäßen Druckschrift ist nun zu entnehmen, dass zwischen wenigstens einem von dorsal gesehen vor der Schwenkachse gelegenen Lagerpunkt und/oder einen ventral gesehen vor der Schwenkachse gelegenen Lagerpunkt und dem Prothesenfuß ein Kraftübertragungselement angeordnet ist, welches den Prothesenfuß beim Beugen des Kniegelenkes aus einer Spitzfußlage oder Mittelfußstellung des Kunstfußes in zunehmendem Maße in die Hackenfußlage überführt. Damit soll die Notwendigkeit, den gesunden natürlichen Fuß in eine unphysiologische Spitzfußstellung zu bringen, damit das künstliche Bein durchpendeln kann, nicht mehr gegeben sein und der Bewegungsablauf natürlicher erscheinen.

Wenn die gattungsgemäße Beinprothese auch schon einen deutlichen Fortschritt gegenüber dem ihr zugrunde liegenden Stand der Technik erreicht hat, vermag sie nicht, ein bionisches Laufbild zu erzeugen, also ein der Natur nachempfundenes Laufbild. Der Grund hierfür ist darin zu sehen, dass die Pendelbeweglichkeit des Prothesenunterschenkels in weiten Bereichen gleich ist. Im natürlichen Bein hingegen verändert sich die Beweglichkeit des Kniegelenkes in Abhängigkeit von seiner Beugeposition. So ist das Knie im gebeugten Zustand, also im Bereich von ca. 90°, relativ frei beweglich. Mit abnehmendem Beugewinkel, das heißt bei Streckung des Beines, wird der Widerstand gegen eine weitere Beugung vergrößert. Im gestreckten Zustand des Beines ist das Kniegelenk de facto steif.

Das Fehlen der veränderlichen Widerstandskräfte gegen eine weitere Beugung des Knies beeinträchtigt bei der Beinprothese gemäß der gattungsgemäßen Druckschrift also das bionische Laufbild.

Die Beinprothese gemäß der DE 199 53 972 A1 weist zwar einen elektrischen Wandler zur Erzeugung eines elektrischen Signals auf, welches dann weiter verarbeitet wird, um das Kniegelenk abhängig vom Beugewinkel abzubremsen. In dem Dokument ist jedoch nur davon die Rede, dass ein Winkelerkennungsmechanismus zum Einsatz kommt. Dies ist darauf zurückzuführen, dass bei dem Kniegelenk lediglich von einem Scharniergelenk die Rede ist, bei dem es keine zusätzlichen translatorischen Bewegungen des Oberteils des Kniegelenks gegenüber dessen Unterteil gibt, wie dies aber bei einem natürlichen Kniegelenk der Fall ist.

Gleiches trifft im Übrigen zu auf die Beinprothese gemäß der US-A-6,113,642, welche ebenfalls nur von einem Scharniergelenk ausgeht, bei dem translatorische Bewegungen des Oberteils des Kniegelenks gegenüber dem Unterteil nicht auftreten.

Vor diesem Hintergrund ist es die Aufgabe der vorliegenden Erfindung, hier Abhilfe zu schaffen, das heißt eine gattungsgemäße Beinprothese so weiterzubilden, dass sie ein bionisches Laufbild gestattet.

Gelöst wird diese Aufgabe dadurch, dass jede Beugeposition des Kniegelenkes durch einen Wandler in ein eineindeutiges elektrisches Signal umgesetzt wird, welches einer programmierbaren Steuerungseinrichtung zugeführt wird, die ein Signal generiert, mit welchem ein elektrisch verstellbarer Aktuator angesteuert wird, der den Widerstand des Kniegelenkes gegen bzw. für eine weitere Beugung dem Signal entsprechend vergrößert bzw. verkleinert.

Es findet also in der erfindungsgemäßen Beinprothese eine elektrische Steuerung des besagten Widerstandes statt. Bei gestrecktem Bein im Bereich von 160° bis 180° wird der Widerstand so hoch eingestellt werden, dass das Bein de facto steif ist. Im Übergangsbereich zwischen 120° und 135° befindet sich das Knie im gesicherten Zustand, lässt jedoch eine Beugebewegung noch zu. Im gebeugten Zustand des Knies ist der Unterschenkel gemäß der natürlichen Vorlage relativ frei beweglich. Voraussetzung für die Steuerung ist die Erfassung der Beugeposition des Kniegelenkes und die eineindeutige Zuordnung eines elektrischen Signals, mit dem die weitere Steuerung erfolgt. Die eindeutige Beugeposition repräsentiert nicht nur den Beugewinkel, sondern auch die translatorische Bewegung des Oberteils des Knies gegenüber dem Unterteil nach hinten bei zunehmender Beugung des Gelenkes. Dies ist die Besonderheit des Kniegelenkes, wie es aus der schon genannten EP-B-0 358 056 bekannt ist. Ein reines Polscharnier, wie es in der EP-B 0 549 855 zum Einsatz kommt, vermag diese Eigenschaften nicht aufzuweisen. Bei einem Kniegelenk mit einem reinen Pol- oder Scharniergelenk ist nämlich die Bedingung, dass sich der Abstand eines dorsal gesehen vor der Schwenkachse gelegenen Punktes zum Ende des Prothesenunterschenkels mit zunehmender Beugung des Kniegelenkes stetig verkleinert, nicht erfüllt. Die so vorgegebene Polkurve würde zwar beispielsweise bei der ersten Bewegung von der Strecklage hin zur Beugelage ebenfalls eine Verringerung des Abstandes mit sich bringen. Nach Erreichen eines Totpunktes aber würde sich der Abstand wieder vergrößern. Dies würde bei einer Beinprothese bei Durchführung der kompletten Bewegung des Kniegelenkes von der Strecklage zur Beugelage dazu führen, dass der Fuß zunächst leicht in die Hackenfußlage geschwenkt wird, nach Überschreiten des besagten Totpunktes aber wieder in die Ausgangslage oder Spitzfußstellung geschwenkt würde, so dass im Ergebnis bei voller Beugung des Kniegelenkes wieder quasi eine Spitzfußstellung des Fußes gegenüber des Prothesenunterschenkels vorläge. Die Generierung eines für jede Beugeposition hin eineindeutiges elektrisches Signal wäre somit nicht möglich.

Gemäß einer bevorzugten Ausführungsform ist es vorgesehen, dass das Kraftübertragungselement aus einer am Kniegelenk und an dem Prothesenfuß angelenkten Schubstange aufgebaut ist. Am proximalen Ende kann die Schubstange beispielsweise im dorsalen Bereich des Kniegelenkes angelenkt sein, um die sich bei der Verringerung des Abstandes des Anlenkpunktes zum Ende des Prothesenunterschenkels für die Ausführung der Schwenkbewegung des Prothesenfußes von der Ausgangslage hin in die gewünschte Hackenfußlage auszunutzen. Besonders zuverlässig wird der Prothesenfuß in die Hackenfußlage bei der Bewegung des Kniegelenkes von der Streck- in die Beugelage bewegt, wenn die Beinprothese gemäß einer vorteilhaften Weiterbildung so ausgebildet ist, dass der Prothesenfuß an den Prothesenunterschenkel um einen ventral gelegenen Schwenkpunkt schwenkbar gekoppelt ist und dass das Kraftübertragungselement an den Prothesenfuß an einem dorsal gelegenen Lagerpunkt angelenkt ist. Das Kraftübertragungselement bewirkt dann die Einleitung eines Drehmomentes um den ventral gelegenen Schwenkpunkt herum, wodurch der Prothesenfuß sicher in die Hackenfußlage schwenkt.

Gemäß einer besonders bevorzugten Ausführungsform wird vorgesehen, dass zwischen dem Prothesenfuß und einem Lager an dem Prothesenunterschenkel ein flexibler, auf seine effektive Länge einstellbarer Zügel gespannt ist, der bei zunehmender Beugung des Kniegelenkes eine zunehmende Lose erhält.

Der Zügel übernimmt im wesentlichen die Aufgabe der natürlichen Achillessehne. Die Hauptaufgabe des Zügels besteht darin, in Streckstellung des Kniegelenkes den Fuß in seine Ausgangslage zurückzubringen. Des weiteren dient der Zügel aufgrund der Einstellmöglichkeit seiner effektiven Länge, beispielsweise durch einen Anschlag mit einem Gewinde, mit welchem das betreffende Ende des Zügels verschraubbar ist, zur individuellen Einstellung einer Spitzfußstellung des Prothesenfußes. Diese Einstellung differiert in der Regel durch die unterschiedlichen Fußabsätze von Patient zu Patient.

In dem Kraftübertragungselement ist vorzugsweise ein Rückstellelement integriert, welches bei Streckung des Knies nach vorheriger Beugung den Prothesenfuß aktiv wieder in seine Ausgangslage verbringt. Dieses aktive Rückstellelement unterstützt die Wirkung des weiter oben erwähnten Zügels bei beginnender Bewegung von der Beugelage in die Strecklage des Knies. Dabei entfaltet in erster Linie das erwähnte Kraftübertragungselement die Rückstellwirkung, wohingegen bei annäherndem Erreichen der Strecklage der Zügel in erster Linie seine Wirkung entfaltet.

Bei der vorerwähnten Weiterbildung ist es bevorzugt, dass das Rückstellelement eine eine Spiralfeder aufnehmende Führungshülse sowie einen in der Hülse geführten Stempel als Teil der Schubstange aufweist, derart, dass bei zunehmender Beugung des Kniegelenkes die Feder zunehmend druckbelastet wird und bei Streckung des Kniegelenkes die Federkraft den Prothesenfuß in die Ausgangsposition verschwenkt. Je stärker also das Knie gebeugt wird, desto höhere Federkräfte werden in dem Rückstellelement erzeugt. Es ist vorteilhaft vorgesehen, dass die Führungshülse in einem an dem Prothesenunterschenkel befestigten Gehäuse gelagert ist. Hierdurch ergibt sich eine kompakte Einheit, die für den Patienten relativ einheitlich zu handhaben ist.

Was nun den elektrisch verstellbaren Aktuator betrifft, so ist dieser besonders bevorzugt als Hydraulikzylinder mit mehreren steuerbaren Ventilen ausgebildet. Das erwähnte, jede Beugeposition des Kniegelenkes eineindeutig repräsentierende elektrische Signal vom Wandler dient nach Weiterverarbeitung in der Steuerungseinrichtung zur Ansteuerung der elektrisch steuerbaren Ventile, die ihrerseits den Fluss der Hydraulikflüssigkeit des Zylinders beeinflussen. Durch die hydraulische Steuerung wird die Kraft, mit welcher der Kolben des Hydraulikzylinders bewegt werden kann, gesteuert.

Andere Aktuatoren als ein Hydraulikzylinder sind denkbar, wie beispielsweise eine Magnetbremse. Entscheidend allein ist, dass der Aktuator den Widerstand des Kniegelenkes für oder gegen eine weitere Beugung steuert. Die vom Aktuator erzeugten Kräfte werden über eine geeignete Mechanik, wie beispielsweise eine Anordnung mindestens eines am Oberteil des Kniegelenkes angreifenden Schubbandes, realisiert.

In Abhängigkeit von der jeweiligen Beugeposition des Knies wird also zum einen der Fuß über das Kraftübertragungselement aus der Spitzfußlage stetig in eine Hackenfußlage verschwenkt und jeder Beugeposition wird ein Widerstand gegen bzw. für eine weitere Beugung des Kniegelenkes zugeordnet. Dies sorgt für den anvisierten bionischen Bewegungsablauf.

Der Wandler, der für jede Beugeposition ein eineindeutiges Signal generieren soll, setzt die Beugebewegung vorzugsweise in eine translatorische Bewegung eines in Führungsleisten geführten Messblockes um, der in einem ihm zugeordneten Sensor das die Beugeposition des Kniegelenkes repräsentierende Signal erzeugt.

Die Umsetzung der komplizierten Bewegung eines Kniegelenkes bei einer Beugebewegung, die eine Roll- und Gleitbewegung darstellt, ist nicht trivial. Die Einsetzung beispielsweise eines Drehpotentiometers um die Beugeachse des Knies führt nicht zu dem erwünschten Ziel, da in dem so generierten Signal keinerlei Anteil der translatorischen Bewegung des Oberteils gegenüber dem Unterteil bei zunehmender Beugung des Kniegelenkes nach hinten repräsentiert ist. Der Messblock steht bevorzugt mit dem mindestens einen Schubband in Verbindung, mit welchem der Aktuator die von ihm erzeugte Kraft in das Kniegelenk leitet.

In die programmierbare Steuerungseinrichtung wird das vom Wandler erzeugte Signal geleitet, welche daraus ein patientenindividuelles Signal generiert. Für jeden Patienten ist individuell ein Bewegungsablauf programmierbar, das heißt jeder Beugeposition des Kniegelenkes kann patientenindividuell eine Widerstandskraft zugeordnet werden, welche vom Aktuator erzeugt wird. Diese nach Art einer Look-Up-Table erzeugte Tabelle wird händisch ermittelt, wobei zum Beispiel die effektive Länge des erwähnten flexiblen Zügels, aber auch der Wider-stand des Aktuators in bestimmten Beugepositionen des Kniegelenkes eingestellt werden kann. Die so ermittelten Werte können dann in der programmierbaren Steuerungseinrichtung beispielsweise mittels eines Laptops abgespeichert werden und stehen dann für den Betrieb bereit.

Besonders bevorzugt ist der Wandler so ausgeführt, dass der Messblock ein Magnet und der ihm zugeordnete Sensor ein Hallsensor ist, an welchen der Magnet, der mit dem Schubband zur Übertragung der Aktuatorkräfte in Verbindung steht, vorbeigeführt wird. Der große Vorteil dieser Anordnung ist, dass sie absolut verschleißfrei ist.

Die Stromversorgung der Steuereinrichtung, des Aktuators und ggf. des Umwandlers kann über in der Prothese angeordnete Batterien oder Akkus erfolgen.

Die Erfindung wird beispielhaft anhand der Zeichnungsfiguren näher erläutert. Hierbei zeigt:
- Fig. 1: die schematische Seitenansicht der vollständigen Beinprothese,

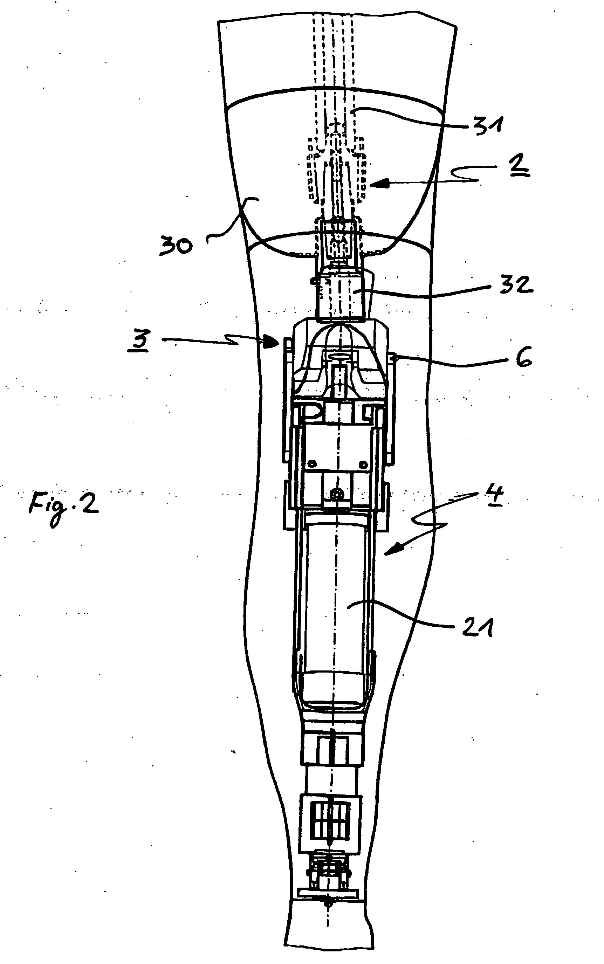

- Fig. 2: die Ansicht der Prothese von ventral ohne Fuß und

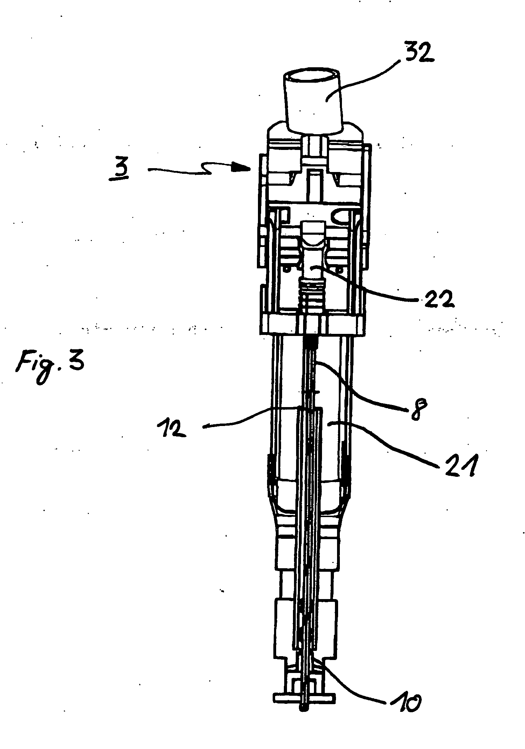

- Fig. 3: die Ansicht des Kniegelenkes und des Prothesenunterschenkels von dorsal.

Nachfolgend sind gleiche Teile mit denselben Bezugszeichen versehen.

Die Beinprothese besteht aus einem Adapter 2, der mit dem Oberschenkelstumpf 30 des Patienten in der Weise verbunden ist, dass der Adapter 2 an den Femurstumpf 31 fixiert ist. An das Ankoppelungselement 32 des Adapters 2 ist ein Kniegelenk 3 gekoppelt, dem sich distal ein Prothesenunterschenkel 4 anschließt, welcher schließlich einen Prothesenfuß 5 trägt, in der Weise, dass der Prothesenfuß 5 mit dem Prothesenunterschenkel 4 schwenkbar verbunden ist.

Das Kniegelenk 3 weist eine Schwenkachse 6 auf, um welche das Oberschenkelteil des Kniegelenkes 3 gegenüber dem Unterschenkelteil 4 schwenkbar ist. Das Kniegelenk 3 hat die spezielle Eigenschaft, dass es bei dem Übergang von der Strecklage hin zur Beugelage um die Schwenkachse 6 eine Roll-Gleitbewegung ausführt. Dies führt dazu, dass sich der Abstand eines dorsal gesehen vor der Schwenkachse 6 gelegenen Punktes D zum Ende 7 des Prothesenunterschenkels 4 stetig verkleinert. Entsprechend vergrößert sich der besagte Abstand bei einem ventral gesehen vor der Schwenkachse 6 gelegenen Punktes V stetig bei Ausführung der Bewegung von der Streck- in die Beugelage des Kniegelenkes 3.

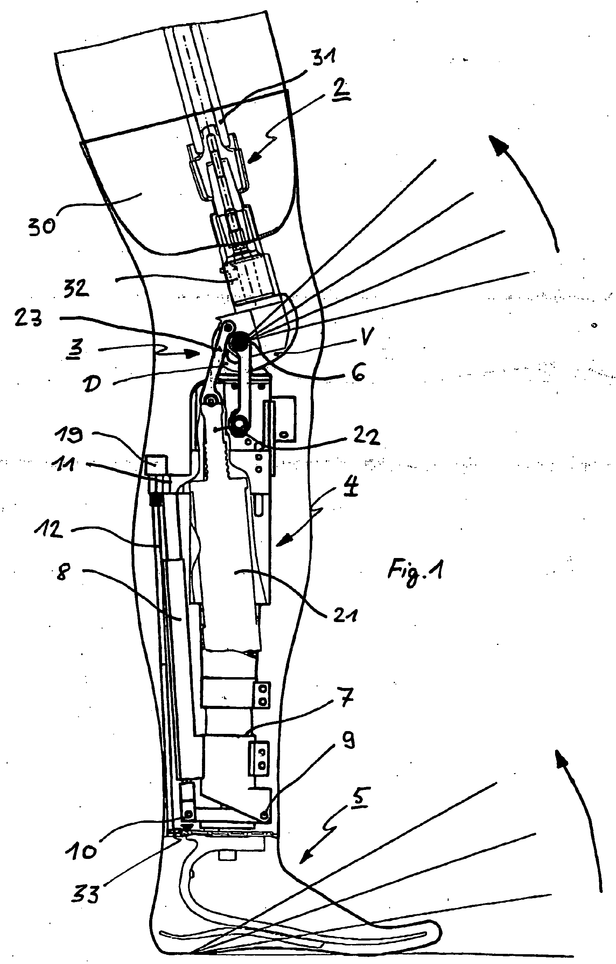

Der Prothesenfuß 5 ist am Ende 7 des Prothesenunterschenkels 4 um einen ventral gelegenen Schwenkpunkt 9 schwenkbar angelenkt. Wird der Prothesenfuß 5 um den Schwenkpunkt 9 geschwenkt, begibt sich der Prothesenfuß 5 in die Hackenfußlage. Dies ist angedeutet in Fig. 1 durch den Strahlenkranz im Fußbereich sowie im Kniebereich. Wird das Knie in Richtung des angedeuteten Pfeils gebeugt, so wird die Fußprothese in Richtung des angedeuteten Pfeils angehoben.

Die Koppelung der Bewegung des Kniegelenkes 3 mit der Bewegung des Prothesenfußes 5 gelingt über das Kraftübertragungselement 8, welches proximal am Kniegelenk 3 angelenkt ist. Die Kraft des Kraft-übertragungselementes 8 wird in das Ansatzstück 33 am Lagerpunkt 10 dorsal eingeleitet. Das Ansatzstück 33 übernimmt hierbei die Funktion des natürlichen oberen Sprunggelenkes.

Darüber hinaus ist bei dem gezeigten Ausführungsbeispiel dorsal zwischen einem an dem Prothesenunterschenkel 4 fixierten Lager 11 und dem Prothesenfuß 5 ein flexibler Zügel 12 gespannt. Der Zügel 12 besteht beispielsweise aus einer flexiblen Stahllitze. Er übernimmt die Funktion der natürlichen Achillessehne. Er dient dazu, beim Übergang von der Beuge- in die Strecklage des Kniegelenkes 3 den Prothesenfuß 5 wieder vollständig in die Ausgangslage zurückzuschwenken. Darüber hinaus dient er der individuellen Einstellung einer Hackenlage des Prothesenfußes 5. Dazu weist der Zügel 12 am proximalen Ende eine Gewindehülse auf, welche mit einem Ansatz 19 mit Innengewinde sowie dem Lager 11 zusammenarbeitet, derart, dass der Ansatz 19 einen Anschlag am Lager 11 bildet. Durch Verschraubung des Ansatzes 19 lässt sich patientenindividuell die Ausgangsfußstellung (Spitzfußstellung) einstellen.

Nicht dargestellt ist ein Rückstellelement in dem Kraftübertragungselement, das bei Streckung des Kniegelenkes 3 nach vorheriger Beugung den Prothesenfuß 5 wieder in seine Ausgangslage zurückdrängt.

In dem Prothesenunterschenkel 4 ist nun ein Hydraulikzylinder 21 mit seiner Kolbenstange 22 angeordnet. Der Hydraulikzylinder 21 dient als Aktuator zur Verstellung des Widerstandes des Kniegelenkes gegen bzw. für eine weitere Beugung. Hierzu wird der Hydraulikzylinder 21 von Mehrkammerventilen (nicht dargestellt) entsprechend mit Hydraulikflüssigkeit versorgt. Die Mehrkammerventile sind elektrisch ansteuerbar, und zwar von einer programmierbaren Steuerungseinrichtung (nicht dargestellt). Diese Steuerungseinrichtung erhält ein Signal von einem Wandler (nicht dargestellt), der die aktuelle Beugeposition des Kniegelenkes 3 in ein eineindeutiges elektrisches Signal umwandelt. Der Beugeposition des Kniegelenkes 3 entsprechend erzeugt der Hydraulikzylinder 21 also eine entsprechende Kraft, die er über seine Kolbenstange 22 weitergibt.

Am Kniegelenk 3 ist dorsal mindestens ein Schubband 23 angebracht, dessen anderes Ende mit der Kolbenstange 22 des Hydraulikzylinders 21 verbunden ist. Über dieses mindestens eine Schubband 23 steuert der Hydraulikzylinder 21 den Widerstand gegen eine weitere Beugung des Kniegelenkes 3.

Mit dem Schubband 23 verbunden ist ein in Führungsleisten geführter Messblock (nicht dargestellt), welcher Teil des Sensors ist. Mit zunehmender Beugung des Kniegelenkes 3 wird der Messblock über das Schubband 23 weiter nach unten verfahren. Dabei streicht er an einem Sensor (nicht dargestellt) vorbei, vorzugsweise an einem Hallsensor, wenn der Messblock ein Magnet ist, wobei ein Signal generiert wird. Dieses Signal repräsentiert in eineindeutiger Weise die jeweilige Beugeposition des Kniegelenkes 3 und wird der schon erwähnten Steuerungseinrichtung zur Weiterverarbeitung zugeführt.

Wesentlich bei der erfindungsgemäßen Beinprothese ist vor allem nicht nur das Vorliegen einer Zwangskoppelung zwischen der Bewegung des Kniegelenkes 3 und der Verschwenkbewegung des Prothesenfußes 5, sondern auch, dass der Widerstand des Kniegelenkes gegen bzw. für eine weitere Beugung entsprechend der jeweiligen Beugeposition des Knies vergrößert bzw. verkleinert wird.

## Patentansprüche

1. Beinprothese zur Adaption an einen Oberschenkelstumpf, bestehend aus einem Adapter (2) für ein Kniegelenk (3), einem daran befestigten Kniegelenk (3) und einem am Kniegelenk (3) angekoppelten Prothesenunterschenkel (4) mit einem daran angelenkten Prothesenfuß (5), der in eine Hackenfußlage schwenkbar ist, wobei das Kniegelenk (3) so ausgebildet ist, dass es beim Übergang von der Strecklage in die Beugelage um eine Schwenkachse (6) eine kombinierte Roll-Gleitbewegung ausführt, derart, dass sich der Abstand eines dorsal gesehen vor der Schwenkachse (6) gelegenen Punktes (D) zum Ende (7) des Prothesenunterschenkels (4) mit zunehmender Beugung stetig verkleinert bzw. eines ventral gesehen vor der Schwenkachse (6) gelegenen Punktes (V) zum Ende (7) des Prothesenunterschenkels (4) stetig vergrößert, wobei zwischen wenigstens einem von dorsal gesehen vor der Schwenkachse (6) gelegenen Lagerpunkt bzw. einem ventral gesehen vor der Schwenkachse (6) gelegenen Lagerpunkt und dem Prothesenfuß (5) ein Kraftübertragungselement (8) angeordnet ist, welches den Prothesenfuß (5) beim Beugen des Kniegelenkes (3) in zunehmendem Maße aus der Spitzfußlage in die Hackenfußlage überführt,
**dadurch gekennzeichnet,**
**dass** jede sowohl den Beugewinkel als auch die translatorische Bewegung des Oberteils des Kniegelenks (3) gegenüber dem Unterteil repräsentierende Beugeposition des Kniegelenkes durch einen Wandler in ein eineindeutiges elektrisches Signal umgesetzt wird, welches einer programmierbaren Steuerungseinrichtung zugeführt wird, die ein Signal generiert, mit welchem ein elektrisch verstellbarer Aktuator angesteuert wird, der den Widerstand des Kniegelenkes gegen bzw. für eine weitere Beugung dem Signal entsprechend vergrößert bzw. verkleinert.

2. Beinprothese nach Anspruch 1, **dadurch gekennzeichnet, dass** das Kraftübertragungselement (8) aus einer am Kniegelenk (3) und an dem Prothesefuß (5) angelenkten Schubstange aufgebaut ist.

3. Beinprothese nach Anspruch 2 oder 3, **dadurch gekennzeichnet, dass** der Prothesenfuß (5) an den Prothesenunterschenkel (4) um einen ventral gelegenen Schwenkpunkt (9) schwenkbar gekoppelt ist und dass das Kraftübertragungselement (8) an den Prothesenfuß (5) an einem dorsal gelegenen Lagerpunkt (10) angelenkt ist.

4. Beinprothese nach einem der Ansprüche 1 bis 3, **dadurch gekennzeichnet, dass** zwischen dem Prothesenfuß (5) und einem Lager (11) an dem Prothesenunterschenkel (4) ein flexibler, auf seine effektive Länge einstellbarer Zügel (12) gespannt ist, der bei zunehmender Beugung des Kniegelenkes (3) eine zunehmende Lose erhält.

5. Beinprothese nach einem der Ansprüche 1 bis 4, **dadurch gekennzeichnet, dass** in dem Kraftübertragungselement (8) ein Rückstellelement integriert ist, welches bei Streckung des Kniegelenkes (3) nach vorheriger Beugung den Prothesenfuß (5) wieder in seine Ausgangslage (Spitzfußlage) verbringt.

6. Beinprothese nach Anspruch 5, **dadurch gekennzeichnet, dass** das Rückstellelement eine eine Spiralfeder aufnehmende Führungshülse sowie einen in der Hülse geführten Stempel als Teil der Schubstange aufweist, derart, dass bei zunehmender Beugung des Kniegelenks (3) die Feder zunehmend druckbelastet wird und bei Streckung des Kniegelenkes (3) die Federkraft den Prothesenfuß (5) in die Ausgangsposition verschwenkt.

7. Beinprothese nach Anspruch 6, **dadurch gekennzeichnet, dass** die Führungshülse in einem an den Prothesenunterschenkel (4) befestigten Gehäuse gelagert ist.

8. Beinprothese nach einem der Ansprüche 1 bis 7, **dadurch gekennzeichnet, dass** der Aktuator ein Hydraulikzylinder mit mehreren steuerbaren Ventilen ist.

9. Beinprothese nach einem der Ansprüche 1 bis 8, **dadurch gekennzeichnet, dass** der Wandler die Beugebewegung umsetzt in eine translatorische Bewegung eines in Führungsleisten geführten Messblockes, der in einem ihm zugeordneten Sensor das die Beugeposition des Kniegelenkes repräsentierende Signal erzeugt.

10. Beinprothese nach Anspruch 9, **dadurch gekennzeichnet, dass** der Messblock ein Magnet und der Sensor ein Hallsensor ist.

## Claims

1. Leg prosthesis for adaptation to a thigh stump consisting of an adapter (2) for a knee joint (3), a knee joint (3) attached thereto and a prosthesis lower leg (4) coupled to the knee joint (3) having a prosthesis foot (5) hinged thereto, which can be pivoted into a club foot position, wherein the knee joint (3) is designed so that it executes a combined rolling and gliding movement during transition from the extended position into the bent position about a pivoting axis (6), such that the distance of a point (D) placed in front of the pivoting axis (6) seen dorsally, to the end (7) of the prosthesis lower leg (4) is reduced continuously with increasing bending, or a point (V) placed in front of the pivoting axis (6) seen ventrally, to the end (7) of the prosthesis lower leg (4) is increased continuously, wherein a force-transmitting element (8), which transfers the prosthesis foot (5) into the club foot position when bending the knee joint (3) to increasing extent from the pointed foot position, is arranged between at least one bearing point placed in front of the pivoting axis (6) seen dorsally, or a bearing point placed in front of the pivoting axis (6) seen ventrally and the prosthesis foot (5), **characterised in that** each bending position of the knee joint representing both the bending angle and the translational movement of the upper part of the knee joint (3) with respect to the lower part is converted into an unambiguous electrical signal by a transducer, which signal is passed to a programmable control device which generates a signal, with which an electrically adjustable actuator is controlled, which increases or decreases the resistance of the knee joint against or for further bending according to the signal.

2. Leg prosthesis according to claim 1, **characterised in that** the force-transmitting element (8) is constructed from a connecting rod hinged to the knee joint (3) and to the prosthesis foot (5).

3. Leg prosthesis according to claim 1 or 2, **characterised in that** the prosthesis foot (5) is coupled pivotably to the prosthesis lower leg (4) about a ventrally placed pivoting point (9) and **in that** the force-transmitting element (8) is hinged to the prosthesis foot (5) at a dorsally placed bearing point (10).

4. Leg prosthesis according to one of claims 1 to 3, **characterised in that** a flexible restraint (12) which can be adjusted over its effective length and which has increasing slackness during increasing bending of the knee joint (3), is clamped between the prosthesis foot (5) and a bearing (11) on the prosthesis lower leg (4).

5. Leg prosthesis according to one of claims 1 to 4, **characterised in that** a restoring element, which brings the prosthesis foot (5) back to its starting position (pointed foot position) during extension of the knee joint (3) after prior bending, is integrated into the force-transmitting element (8).

6. Leg prosthesis according to claim 5, **characterised in that** the restoring element has a guide sleeve accommodating a helical spring and a ram guided in the sleeve as part of the connecting rod, such that the spring is compressively stressed increasingly during increasing bending of the knee joint (3) and the spring force pivots the prosthesis foot (5) into the starting position during extension of the knee joint (3).

7. Leg prosthesis according to claim 6, **characterised in that** the guide sleeve is mounted in a housing attached to the prosthesis lower leg (4).

8. Leg prosthesis according to one of claims 1 to 7, **characterised in that** the actuator is a hydraulic cylinder having several controllable valves.

9. Leg prosthesis according to one of claims 1 to 8, **characterised in that** the transducer converts the bending movement into a translational movement of a measuring block guided in guide strips, which measuring block produces the signal representing the bending position of the knee joint in a sensor assigned to it.

10. Leg prosthesis according to claim 9, **characterised in that** the measuring block is a magnet and the sensor a Hall sensor.

## Revendications

1. Prothèse de jambe pour adaptation sur un moignon de cuisse, constituée d'un adaptateur (2) pour une articulation de genou (3), d'une articulation de genou (3) qui y est fixée et d'une jambe de prothèse (4) couplée à l'articulation de genou (3) avec un pied de prothèse (5) articulé sur celle-ci, qui peut pivoter dans une position de pied bot talus, l'articulation de genou (3) étant conformée de sorte que, lors du passage de la position d'extension à la position fléchie autour d'un axe pivot (6), elle effectue un mouvement roulant-glissant combiné de manière que la distance d'un point (D) situé, vu de dos, devant l'axe pivot (6) à l'extrémité (7) de la jambe de prothèse (4) soit constamment réduite à mesure que le fléchissement augmente ou selon le cas que la distance d'un point (V) situé, vu de face, devant l'axe pivot (6) à l'extrémité (7) de la jambe de prothèse (4) soit constamment agrandie, dans laquelle est agencé, entre au moins un point d'appui situé, vu de dos, devant l'axe pivot (6) ou selon le cas un point d'appui situé, vu de face, devant l'axe pivot (6) et le pied de prothèse (5), un élément de transfert de forces (8) qui transfère le pied de prothèse (5), lors du fléchissement de l'articulation de genou (3), dans une mesure croissante de la position de pied bot équin à la position de pied bot talus, **caractérisée en ce que** toute position de fléchissement de l'articulation du genou représentant autant l'angle de fléchissement que le mouvement de translation de la partie supérieure de l'articulation de genou (3) par rapport à la partie inférieure est transformée par un convertisseur en un signal électrique univoque, qui est acheminé à un dispositif de commande programmable, qui génère un signal par lequel est commandé un dispositif d'actionnement réglable électriquement qui augmente ou réduit la résistance de l'articulation du genou contre ou pour un autre fléchissement en fonction du signal.

2. Prothèse de jambe selon la revendication 1, **caractérisée en ce que** l'élément de transfert de forces (8) est constitué d'une bielle articulée sur l'articulation de genou (3) et sur le pied de prothèse (5).

3. Prothèse de jambe selon la revendication 1 ou 2, **caractérisée en ce que** le pied de prothèse (5) est couplé à la jambe de prothèse (4) de manière à pouvoir pivoter autour d'un point pivot (9) situé sur le devant et **en ce que** l'élément de transfert de forces (8) est articulé sur le pied de prothèse (5) en un point d'appui (10) situé sur le dos.

4. Prothèse de jambe selon l'une quelconque des revendications 1 à 3, **caractérisée en ce qu'**une bride (12) souple réglable à sa longueur efficace est tendue entre le pied de prothèse (5) et un palier (11) sur la jambe de prothèse (4), ladite bride recevant à mesure du fléchissement de l'articulation de genou (3) une mobilité croissante.

5. Prothèse de jambe selon l'une quelconque des revendications 1 à 4, **caractérisée en ce qu'**à l'élément de transfert de forces (8) est intégré un élément de rappel qui, lors de l'extension de l'articulation de genou (3) après un fléchissement précédent, ramène le pied de prothèse (5) dans sa position de départ (position de pied bot équin).

6. Prothèse de jambe selon la revendication 5, **caractérisée en ce que** l'élément de rappel présente une douille de guidage logeant un ressort spiralé ainsi qu'un poinçon guidé dans la douille comme partie de la bielle de manière qu'à mesure que le fléchissement de l'articulation de genou (3) augmente, le ressort soit de plus en plus soumis à une pression et **en ce que** la force élastique fait pivoter le pied de prothèse (5) par extension de l'articulation de genou (3) dans la position de départ.

7. Prothèse de jambe selon la revendication 6, **caractérisée en ce que** la douille de guidage est logée dans un boîtier fixé à la jambe de prothèse (4).

8. Prothèse de jambe selon l'une quelconque des revendications 1 à 7, **caractérisée en ce que** l'élément d'actionnement est un cylindre hydraulique avec plusieurs soupapes qui peuvent être commandées.

9. Prothèse de jambe selon l'une quelconque des revendications 1 à 8, **caractérisée en ce que** le convertisseur transforme le mouvement de fléchissement en un mouvement de translation d'un bloc de mesure guidé dans des glissières de guidage et qui, dans un capteur qui lui est affecté, génère le signal représentant la position de fléchissement de l'articulation du genou.

10. Prothèse de jambe selon la revendication 9, **caractérisée en ce que** le bloc de mesure est un aimant et **en ce que** le capteur est un capteur à effet Hall.
